# EUROPEAN PATENT APPLICATION

(11) **EP 3 842 031 A1**
(43) Date of publication of application: **30.06.2021**
(21) Application number: 20216758.1
(22) Date of filing: 22.12.2020
(51) Int. Cl.: A61K 9/00, A61K 31/137, A61K 31/56, A61K 45/06, A61P 11/06

(54) **NEW THERAPEUTIC INHALATION SYSTEM PROVIDING REDUCED DOSE OF FLUTICASONE PROPIONATE AND SALMETEROL XINAFOATE WITH IMPROVED EFFICACY AND SAFETY PROFILE**

(30) Priority: 23.12.2019 PL 43237419
(71) Applicant: PRZEDSIEBIORSTWO FARMACEUTYCZNE LEK-AM SP Z O. O., 05-170 Zakroczym (PL)
(72) Inventor: HEJDUK, Arkadiusz, 05-462 Wiazowna (PL); URBANSKA, Agnieszka, 05-110 Jablonna (PL); OSINSKI, Andrzej, 00-875 Warszawa (PL); LUKASZEWICZ, Piotr, 01-401 Warszawa (PL); DOMANSKI, Maciej, 09-100 Plonsk (PL)
(74) Representative: BRANDPAT Patent and Trademark Attorneys Chlebicka Czyz Galazkiewicz Ziolkowski Professional Partnership

(57) **Abstract**

A pharmaceutical composition in the form of dry powder for inhalation comprising combination of two active pharmaceutical ingredients fluticasone propionate and salmeterol xinafoate for use in a method of treating asthma and chronic obstructive pulmonary disease (COPD), wherein particle size distribution of fluticasone propionate for d90 value is less than 10 µm, and particle size distribution of salmeterol xinafoate for d90 value is less than 10 µm, for administration in a dose of 250/25 µg or 125/25 µg with a capsule inhaler having intrinsic aerodynamic resistance *R_{D}* in the range from 0.01 hPa^{0,5} min dm⁻³ to 0,2 hPa^{0,5} min dm⁻³. The composition in the dose of 250/25 µg is bioequivalent to the dose of 500/50 µg of reference medicinal product.

## Description

The present invention relates to a dry powder for inhalation formulation comprising fluticasone propionate and salmeterol xinafoate.

### Technical field

The treatment of asthma and COPD globally is dominated by the use of inhaled fixed-dose combinations of a long-acting β₂-agonist (LABA) and an inhaled corticosteroid (ICS) (Page C, Cazzola M. 2014;). Asthma affects about 7% of the European population, and its prevalence appears to be rising. Bronchial asthma is a chronic disease characterized by variable airway narrowing causing airflow obstruction. The treatment of asthma is internationally agreed upon and guidelines have been developed for the management of asthma. All guidelines focus on the treatment of inflammation and recommend inhaled corticosteroids for all adults and children except those with mild, intermittent disease. Management should take into account that asthma is a condition associated with the following: airway obstruction that can be prevented or reversed by bronchodilators, exacerbations caused by chronic inflammation which can be prevented or reversed by anti-inflammatory drugs.

Salmeterol is a potent and highly selective long acting β2-adrenoceptor agonist. It has a long duration of action when inhaled. Stimulation of β2-membrane receptor increases intracellular cAMP levels, which activates protein kinases responsible for airway smooth muscle relaxation. Additional effects are: an inhibition of inflammatory mediator release from mast cells, a reduction of micro-vascular leakage, an increase in mucocilliary transport (Johnson and Hagan 2001) and a modulation of tachykinins' release from airway sensory nerves (Verleden, Belvisi et al. 1993).

In asthmatic patients, the addition of salmeterol to low-dose inhaled steroids decreases the number of eosinophils and reduces mast cells and CD4+ T cells in airway tissue, and significantly inhibits the degree of angiogenesis occurring as part of the remodelling process. These effects were not observed with higher doses of GCS and may contribute to the increase of clinical efficacy with long acting β2-adrenoceptor agonist/steroid combination therapy.

Fluticasone propionate is a corticosteroid that treats airway inflammation in asthma. It has potent anti-inflammatory properties, inhibits the production and release of a variety of inflammatory mediators, enhances the synthesis of anti-inflammatory proteins and accelerates the apoptosis of inflammatory cells (lymphocytes, eosinophils and mast cells).

Fluticasone propionate acts by way of a cytoplasmatic glucocorticoid receptor (GR), to which it attaches after passing through the cell membrane. The activated complex then moves to the nucleus and binds to DNA. This induces the synthesis of anti-inflammatory factors. Mak et al. observed that the activation of GR increased β2-receptor mRNA levels and the number of β2-receptors in human peripheral lung in vitro, by increasing the rate of β2-receptor gene transcription. Fluticasone propionate is a highly potent inhaled corticosteroid with an oral bioavailability of less than 1 percent and twice the potency of the beclomethasone dipropionate in the control of chronic asthma. Fluticasone propionate also compares favourably with other inhaled corticosteroids in patients with all severities of asthma.

Clinical research has confirmed its efficacy in patients with mild to moderate asthma, and also in patients with severe disease and in children. Results from clinical trials with concurrent ICS and salmeterol have consistently shown that the use of these two types of anti-asthmatic agents together provides better asthma control and lower rates of exacerbations compared with the use of higher doses of ICS alone (Shrewsbury, Pyke et al. 2000, Matz, Emmett et al. 2001, Nelson, Chapman et al. 2003, Reynolds, Lyseng-Williamson et al. 2005, Gibson, Powell et al. 2007, Wang, Skrepnek et al. 2008). Recent studies have also shown that treatment with a low dose of the combination product is as effective as a higher dose of an ICS in controlling airway inflammation and is ICS sparing (Busse, Koenig et al. 2003, Jarjour, Wilson et al. 2006). The introduction of combination product delivered by the hydrofluoroalkane (HFA) metered dose inhaler (MDI) for use in adults and adolescents has also been shown to be safe and effective (Nelson, Wolfe et al. 2003).

Inhalers are well known and widely used devices for administering pharmaceutical products to the respiratory tract by inhalation. The capsule inhaler is equipped with two needles. Capsules for powder inhalers generally comprise hard gelatins or cellulose derivatives. In the area of powder inhalers, single-dose and multidose devices are known. In single-dose powder inhalers, dosing can be in the form of capsules containing a powder formulation. Powder formulations contain the active substances in micronized form (with a particle size of approx. 1-5 µm), and one or more excipients, generally lactose.

Dry powder inhalers (DPIs) are personal dosimetric inhalation devices with a wide possibility to deliver to the lungs a variety of therapeutic compounds (Frijlink and de Boer, 2004). Since in all DPIs powder has to be suspended in the air just before inhalation, the effective aerosolization process is the main technical challenge of their construction and use. The word 'effective' is used here to stress that aerosol released from a DPI must have special properties, i.e. it should be characterized by the required particle size distribution and the reproducibility of inhaled dose. It is agreed that the mass of particles with the aerodynamic diameter below 5 µm ('fine particles') should be maximized to assure the penetration of inhaled medicine to the deep bronchial tree, i.e. to reach their desired sites of therapeutic action (Sosnowski, 2016).

In general DPIs can be 'active' or 'passive' devices (Islam and Gladki, 2008). Passive devices, which are the most common class of DPIs, use the energy of inspiratory flow generated by patient to create aerosol from a dose of powder. Obviously, inspiration force and dynamics are not the same in various groups of patients, so the quality of aerosol released from such devices must be less reproducible, and many of such DPIs are flow-dependent (Weuthen et al., 2002). Less common are active DPIs which use external energy sources for converting powder to aerosol. These devices usually utilize compressed air or electric energy (White et al., 2005; Canonica et al., 2015, Han et al, 2002; Hoppentocht et al, 2014) and they have a more complicated construction than passive DPIs. Their main advantage is that the quality of emitted aerosol (i.e. inhaled dose and particle size) should be independent on the inhaling force.

The problem of aerosol production from powders is based on technical factors related to product design: powder quality (drug formulation) and DPI construction, but also on the human factor (i.e. the way of using a given DPI by patients). Also both technical factors (inhaler design and powder properties) cannot be considered as independent, although each is designed separately using a different methodological approach. It can be expected that powder formulation which was originally optimized with a selected DPI will be not similarly good when used with an inhaler with different construction and/or internal aerodynamic resistance. (Hejduk et al., 2018). It is critical to be aware of the above problems during the designing process of both new and generic dry-powder inhalation products. All these factors are highly variable, which potentially leads to very different properties of inhaled aerosol. These factors are not independent, e.g. a correlation exists between inhaler design, because some inhaling devices may be more difficult to use than others. Some construction elements are necessary to obtain aerosol characterized by a high fraction of fine particles. The aerosolization process requires high aerodynamic stresses which can be achieved through some special geometric arrangements for acceleration of the aerosol flow and promotion of turbulence (Gac et al, 2008; Sosnowski et al, 2014).

Active materials commonly delivered by inhalation include bronchodilators such as β2 agonists and anticholinergics, corticosteroids, anti-allergies and other materials that may be efficiently administered by inhalation, thus increasing the therapeutic index and reducing side effects of the active material.

In the regular treatment of asthma, where the use of a combination product (long acting β2-agonist and inhaled corticosteroid) is appropriate, reference medicinal product Seretide® Diskus® inhalation powder in pre-dispensed form is indicated. This reference product comprises inhaler with fluticasone propionate and salmeterol xinafoate in pharmaceutical dosage form of dry powder for inhalation.

### Object of the invention

It is the object of the invention to provide a pharmaceutical composition of two active pharmaceutical ingredients: fluticasone propionate and salmeterol xinafoate in pharmaceutical dosage form of dry powder for inhalation in an inhaler, wherein the dose of 250/25 µg is bioequivalent to the dose of 500/50 µg of reference medicinal product. The object of the invention is to provide a product which can be used in smaller doses of active substances, thus providing less adverse effects, but is equally effective as the reference medicinal product.

### Essence of the invention

A pharmaceutical composition according to the invention is in the form of dry powder for inhalation and comprises combination of two active pharmaceutical ingredients of fluticasone propionate and salmeterol xinafoate, wherein fine particle fraction (FPF) of both active substances, measured at nominal flow in a capsule inhaler having intrinsic aerodynamic resistance *R_{D}* in the range from 0.01 hPa^{0,5} min dm⁻³ to 0.2 hPa^{0,5} min dm⁻³, is in the range of 28 - 35 %, and the composition in the dose of 250/25 µg is bioequivalent to the dose of 500/50 µg of reference medicinal product - Seretide® Diskus® 500 µg /50 µg / dose inhalation powder (denoted as reference product 500/50 µg).

The pharmaceutical composition has lactose as a carrier for active pharmaceutical ingredients. Preferably, the lactose is in the form of monohydrate and preferably particle size distribution of lactose mixture for d90 value is less than 250 µm.

The powder for inhalation is placed in hard capsules, made of gelatin or cellulose derivatives, preferably hydroxypropyl methylcellulose (HPMC) as the main component.
The pharmaceutical composition comprises not more than 1,5 % by weight of salmeterol xinafoate in total mass powder, and not more than 10 % by weight of fluticasone propionate in total mass powder, and not less than 90 % by weight of carrier in total mass powder. Particle size distribution of salmeterol xinafoate for d90 value is less than 10 µm, preferably less than 5 µm and particle size distribution of fluticasone propionate for d90 value is less than 10 µm, preferably less than 5 µm. The pharmaceutical composition is obtained in manufacturing process comprising the stage of mixing the active pharmaceutical ingredients and carrier in a High Shear Mixer.
The obtained powder is to be administered by a capsule inhaler as a dosing device and is encapsulated in hard capsules of size 2, 3 or 4, preferably of size 3. The main component of the capsule is gelatin or cellulose derivative(s), preferably hydroxypropyl methylcellulose (HPMC).

The invention provides the medicinal product comprising the composition defined above in the capsule inhaler having intrinsic aerodynamic resistance *R_{D}* in the range from 0.01 hPa^{0,5}min dm⁻³ to 0,2 hPa^{0,5}min dm⁻³, preferably from 0.05 hPa^{0,5}min dm⁻³ to 0,15 hPa^{0,5} min dm⁻³.

The present invention provides a dry powder inhaler comprising a dry powder medicament comprising fluticasone propionate, salmeterol as xinafoate and lactose carrier in a capsule, wherein the strength 250/25 µg of the medicament (denoted as tested product 250+25 µg) is bioequivalent to the strength 500/50 µg of reference medicinal product - Seretide® Diskus® 500 µg /50 µg / dose inhalation powder (denoted as reference product 500+50 µg). The formulation of the present invention has lactose as the carrier preferably in the form of monohydrate with particle size distribution of lactose mixture for d90 value of less than 250 µm. Lactose occur as white to off-white, odourless, crystalline particles or powder with sweet taste. It is widely used as a diluent in tablets and capsules. Lactose is also one of the most commonly used excipient in formulation of inhalation powder. Since various lactose grades are commercially available that have different physical properties, after the selection of the most suitable material for this particular application the selection of carrier material Lactose monohydrate was restricted to a special lactose grade of d90 value less than 250 µm which grade combines good flow properties with a reproducible particle size distribution, as well as suitable physicochemical characteristics. In this context, compatibility of the lactose monohydrate with Salmeterol Xinafoate and Fluticasone Propionate and batch-to-batch reproducibility of the carrier were shown to be guaranteed.

The powder for inhalation is placed in hard capsules, made of gelatin or cellulose derivatives, preferably made of hydroxypropyl methylcellulose (HPMC). Hydroxypropyl methylcellulose is compatible in applied formulation. Moreover, obtained results show that HPMC capsules are superior to gelatin capsules. Furthermore, the most important property of the capsule for use in a dry powder inhalator (DPI) is its ability to be cut or punctured in a reproducible manner to enable the powder to be emptied from it as completely as possible. The challenge in this process is to ensure that a minimum amount of capsule shell is broken off during cutting or puncturing. Studies comparing gelatin and HPMC capsules have shown the superior performance of HPMC over gelatin in the production of these fragments, particularly after storage at lower relative humidity (Birchall, J.C. et al. 2008)

The pharmaceutical composition for use in the capsule, for the best performance comprises not more than 1,5 % by weight of salmeterol xinafoate in total mass powder, and not more than 10 % by weight of fluticasone propionate in total mass powder, and not less than 90 % by weight of the carrier in total mass powder. Particle size distribution of salmeterol xinafoate for d90 value is less than 10 µm, preferably less than 5 µm and particle size distribution particle size distribution of fluticasone propionate for d90 value is less than 10 µm, preferably less than 5 µm. The particle size distribution of active pharmaceutical ingredients (APIs) is a critical parameter for performance of drug product in the form of dry powder for inhalation. The inventors made a series of experiments to control particle size distribution of active pharmaceutical ingredients and decided on selection of the above specified particle size distribution values of salmeterol xinafoate and fluticasone propionate, which allow to achieve high lung deposition. The pharmaceutical composition as above is obtained in manufacturing process comprising mixing the active pharmaceutical ingredients and carrier in High Shear Mixer, preferably for mixing time from 10 to 60 minutes.. The obtained powder is then encapsulated into hard hydroxypropyl methylcellulose (HPMC) capsules. The capsules filled with inhalation powder are packed into OPA/Alu/PVC//Alu blisters. Furthermore, the process validation in GMP conditions was conducted on three commercial scale batches (500 000 units), for each strength to assure finished product quality, reproducibility and efficacy of the manufacturing process. The high quality of the pharmaceutical composition according to the invention was confirmed in stability studies (in accordance with ICH requirements) conducted for both strengths of tested product.

The capsule inhaler having intrinsic aerodynamic resistance (*R_{D}*) in the range from 0.01 hPa^{0,5}min dm⁻³ to 0,2 hPa^{0,5}min dm⁻³, preferably from 0.05 hPa^{0,5}min dm⁻³ to 0,15 hPa^{0,5} min dm⁻³ is preferred to be used with the capsules comprising the composition of the invention to obtain the best inhalation system.

An innovative approach was used to achieve the bioequivalence with the reference medicinal product, which was focused on optimal deposition of the inhalation powder in patient's respiratory track. Therefore, the throat deposition was reduced to minimize adverse reactions associated with Esophageal candidiasis and lower systemic effects, whereas the lung deposition which is responsible for therapeutic effect was increased. As a result, the composition of the invention with reduced dose, together with the inhaler with specific air resistance (preferably from 0.05 hPa^{0,5} min dm⁻³ to 0,15 hPa^{0,5} min dm⁻³) has better deposition characteristic.

The inhalation device used in the present invention is a mono-dose capsule inhaler. The selected inhalation device commonly known as Cyclohaler has been commercially available for more than 20 years. Essential improvements have been introduced over years. Major improvements implemented into the device include: reduction of overall dimensions, weight reduction, reduction of number of its components, improvement of the perforating system, aesthetics redesign. Some millions of inhalers have been manufactured to date without serious incidents and thus the device can be considered robust and reliable. The selected inhaler provides the optimal aerodynamic particle size distribution (aerosolization performance).

It should be highlighted that inhaler air resistance has been carefully selected in terms of *in vitro* and *in vivo* results as well. It was surprisingly found that the lower resistance of inhaler (which might be subjectively felt by patients with severe or even moderate asthma as its positive feature), the lower lung deposition and lower APIs plasma concentration. On the other hand, higher air resistance of inhalation device gives an opposite effect, which might be explained by the fact, that increased inhaler resistance restricts peak inspiratory flow (PIF) but simultaneously allows a longer inhalation time. Accordingly, low inhaler resistance helps to obtain high airflow rate but at the cost of reduced inhalation time. Hejduk et al. (2018) showed, that independently of inhaler resistance, PIF rate is obtained after approximately 1 sec from the start of inhalation. Therefore, if aerosol is completely released at PIF, then a longer time for aerosol penetration to the lungs during inhalation is available when high-resistant DPIs are used. The achieved PIF is always higher when the inhaler resistance is lower. Inhaler with the higher resistance causes a longer inhalation time. This long emission time is related to a low flowrate through the device, which causes slower rotation of a capsule and slower evacuation of encapsulated powder. It is seen that the real inhalation time with high resistance inhaler can be long enough for the complete emission of the aerosol, however if this time is shorter, the aerosol will have not enough time to penetrate to deep lungs before the exhalation phase begins (Sosnowski et al. 2016). However, too high resistance and in consequence low flowrate significantly impedes inhalation technic by patients. Therefore, there is a tiny space for efficient drug administration, which combines both patients friendly device and APIs adequate size of particles to obtain appropriate lung deposition.

Powder formulation was optimized to obtain adequate aerodynamic particle size distribution used with the inhaler with proper internal aerodynamic resistance. The right lung deposition was achieved after establishing appropriate in vitro - in vivo correlation (IVIVC). The results of pharmacokinetic study confirmed establishing of appropriate IVIVC between the tested product in a dose 250+25 µg and the reference product with a dose 500+50 µg.

### Brief description of drawings

The invention is further illustrated in the drawings, in which:
Fig. 1 presents FPF comparison between tested product 125+25 µg and reference product 250+50 µg;
Fig. 2 presents FPF comparison between tested product 250+25 µg and reference product 500+50 µg;
Fig. 3 presents mouth-throat deposition of salmeterol in tested product 125+25 µg vs. reference product 250+50 µg;
Fig. 4 presents mouth-throat deposition of fluticasone propionate in tested product 125+25 µg vs. reference product 250+50 µg;
Fig. 5 presents mouth-throat deposition of salmeterol in tested product 250+25 µg vs. reference product 500+50 µg;
Fig. 6 presents mouth-throat deposition of fluticasone propionate in tested product 250+25 µg vs. reference product 500+50 µg;

The present invention demonstrates superior (improved) deposition characteristic in relation to therapeutic fraction and has better effectiveness in comparison with commercialized, reference product Seretide® Diskus®. The results for present invention in strength 250/25 µg (tested product 250+25 µg) and Seretide Diskus in strength 500/50 µg (reference product 500+50 µg) from pharmacokinetic study confirmed the equivalence between both products.

The table below shows results from pharmacokinetic study for tested product 250+25 µg and reference product 500+50 µg expressed in Ratio of Geometric Means for AUCt T/R, Cmax T/R and Tmax T/R.

**AUC - area under the plasma concentration curve, Cmax - maximum plasma concentration, Tmax - time until Cmax is reached**

| Clinical trial pharmacokinetic results expressed in Ratio of Geometric Means for T/R between tested product 250+25 µg (T) and reference product 500+50 µg (R) | | | | |
|---|---|---|---|---|
| Active substance | AUCt T/R [%] | Cmax T/R [%] | Tmax T/R [%] | Results for PK parame t ers |
| Salmeterol | 92 | 109 | 100 | Positive |
| Fluticasone propionate | 91 | 93 | 100 | Positive |

Acceptance range of the ratio of the geometric means (test/reference) 80-125% for pharmacokinetic parameters was fulfilled for formulation of tested product used in clinical study (requirements according to Guideline on the Investigation of Bioequivalence CPMP/EWP/QWP/1401/98 Rev. 1 /Corr**). The positive results of the clinical trial (PK) confirmed the appropriate composition of the tested product 250µg/25µg/dose (tested product 250+25 µg ) and the bioequivalence with Seretide® Diskus® 500µg/50µg/dose (reference product 500+50 µg). The positive results from pharmacokinetic studies proved similar pulmonary absorption (for assessment of pulmonary deposition and efficacy) and total systemic exposure (for assessment of safety) (CPMP/EWP/4151/00 Rev. 1).

Furthermore, the inventors have surprisingly found that intra-subject variability in pharmacokinetic study showed higher variability for the reference product 500+50 µg. The lower intra-subject variability during PK studies is the evident and strong advantage of the tested 250+25 µg formulation and provides greater uniformity of dosing by patients than Seretide® Diskus®. Results of lower intra subject variability (CV) for Cmax and AUCt presented in the table below confirm superior characteristic of the tested product over the reference product in terms of uniformity of dosing.

Comparison of intra-subject variability (CV) for Cmax and AUCt in pharmacokinetic study:

| **PK parameter** | **API** | **Tested product 250+25 µg CV** | **Reference product 500+50 µg CV** |
|---|---|---|---|
| Cmax | Salmeterol | 40.47 | 57.54 |
| | Fluticasone propionate | 31.66 | 35.12 |
| AUCt | Salmeterol | 23.73 | 28.49 |
| | Fluticasone propionate | 33.24 | 39.97 |

Moreover, the present invention, based on reduced dose and inhaler with optimized air resistance has demonstrated an improved, beneficial deposition characteristic, which means the higher ratio of therapeutic fraction to emitted dose of the tested product. The present formulation shows superior (improved) deposition characteristic in relation to therapeutic fraction and has better effectiveness than Seretide Diskus.

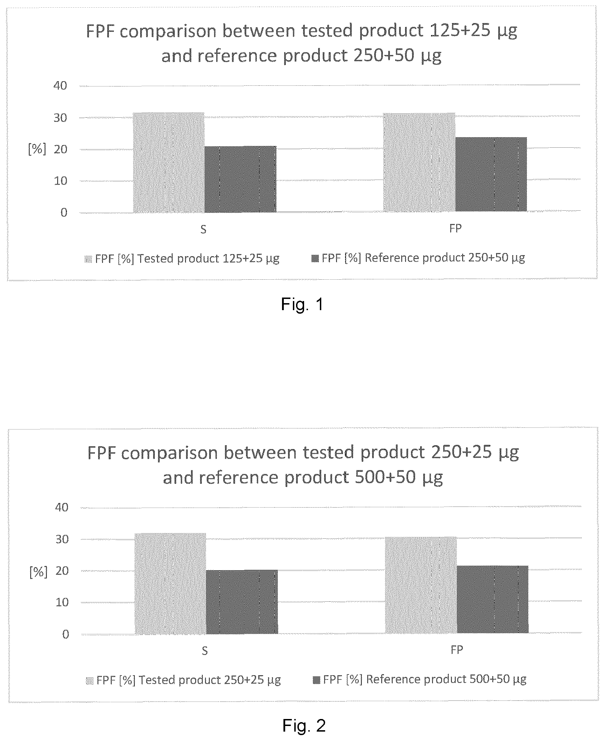

The composition has also improved efficacy in terms of Fine Particle Fraction (FPF) (0-5 µm). The higher FPF [%] values for the tested product in comparison with the reference product confirms improved efficacy, as presented in Fig. 1 and Fig. 2. The results are presented in tables below.

| **FPF comparison between tested product 125+25 µg and reference product 250+50 µg** | | |
|---|---|---|
| **Parameter** | **FPF [%]** | |
| Product | Tested product 125+25 µg | Reference product 250+50 µg |
| **S** | **31.6** | **20.8** |
| **FP** | **31.2** | **23.4** |

| | | |
|---|---|---|
| S - salmeterol, FP - fluticasone propionate, FPF - fine particle fraction. Reference product 250+50 µg, Mean for 3 batches, n = 3x10 = 30 (Air flow rate: 82 L/min). Tested product 125+25 µg, Mean for 3 batches, n = 3x10 = 30 (Air flow rate: 60 L/min). | | |

| **FPF comparison between tested product 250+25 µg and reference product 500+50 µg** | | |
|---|---|---|
| **Parameter** | **FPF [%]** | |
| Product | Tested product 250+25 µg | Reference product 500+50 µg |
| **S** | **31.9** | **20.1** |
| **FP** | **30.5** | **21.2** |

| | | |
|---|---|---|
| S - salmeterol, FP - fluticasone propionate, FPF - fine particle fraction. Reference product 500+50 µg, Mean for 3 batches, n = 3x10 = 30 (Air flow rate: 82 L/min), Tested product 250+25 µg, Mean for 3 batches, n = 3x10 = 30 (Air flow rate: 60L/min), | | |

Aerodynamic particle size distribution (deposition) of the test product for all air flows showed additional superior characteristic.

The composition of the invention, with reduced dose of active substances, might be also safer for patients because of smaller amount of fraction which is deposited in the mouth and throat stages. Lower deposition values on the mouth-throat impactor stages (IP - Induction Port, P - Preseparator) in comparison to the reference medicinal product indicates that the tested product might be considered as providing less adverse effects. Especially inhaled glucocorticoids which may cause in the mouth and throat such adverse effects as oral candidiasis, hoarseness, perioral dermatitis, tongue hypertrophy (Roland NJ at al., 2004), in lower amounts in the mouth and throat are safer for the patients. Salmeterol, as long acting β2-adrenoceptor agonist, might cause such undesirable systemic effects as tachycardia, tremor, throat irritation, and hoarseness/dysphonia. Additionally, mouth-throat fraction might be absorbed from gastrointestinal tract and might cause more undesirable side effects.

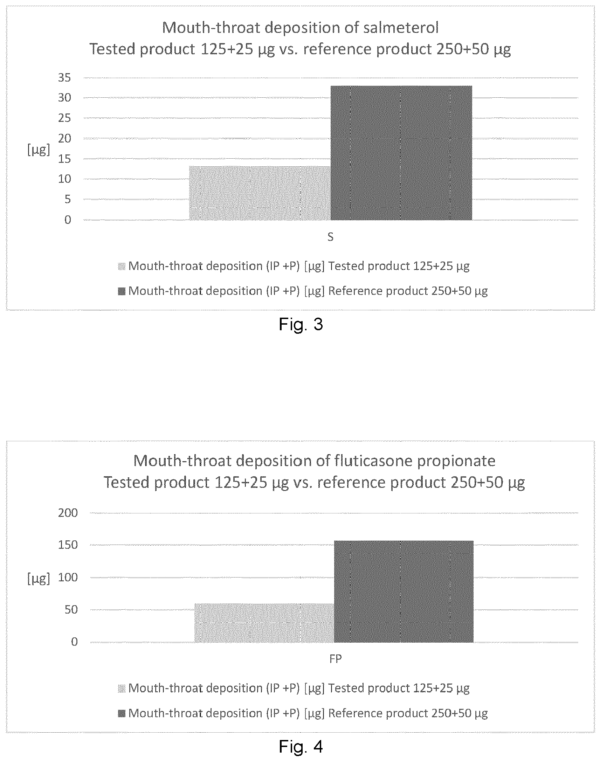

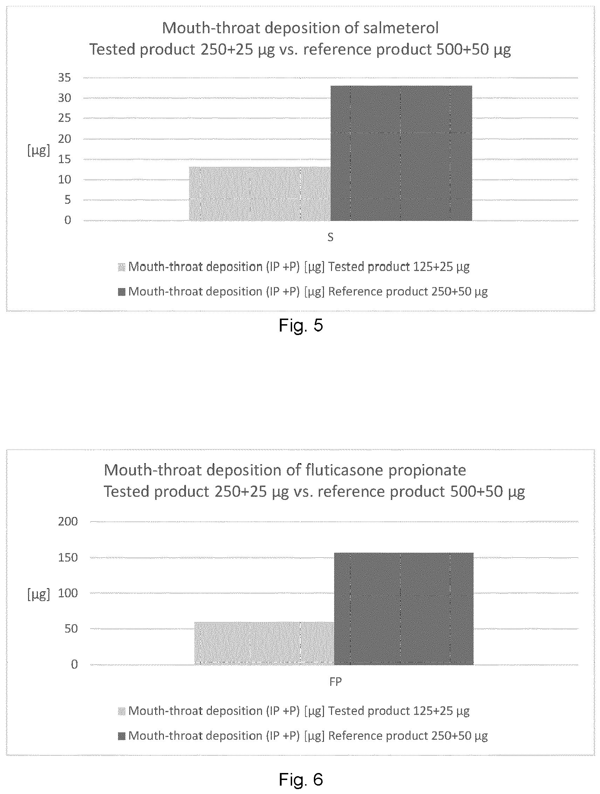

Therefore, the present invention provides the composition with lower unfavorable systemic exposure which allows to minimalize side effects of the drug. Significantly and advantageously, the lower mouth-throat deposition results of the tested product in comparison with the reference product were observed. Results of mouth-throat deposition of salmeterol and fluticasone propionate for both products are presented in tables below and illustrated by Fig. 3-6.

| **Mouth-throat deposition of salmeterol** | | |
|---|---|---|
| **Comparison between tested product 125+25 µg and reference product 250+50 µg** | | |
| **Parameter** | **Mouth-throat deposition (IP +P) [µg]** | |
| Product | Tested product 125+25 µg | Reference product 250+50 µg |
| S | 13.2 | 33.0 |

| | | |
|---|---|---|
| Reference product 250+50 µg, Mean for 3 batches, n = 3x10 = 30 (Air flow rate: 82 L/min), Tested product 125+25 µg, Mean for 3 batches, n = 3x10 = 30 (Air flow rate: 60 L/min), S - salmeterol, IP - Induction Port, P - Preseparator | | |

| **Mouth-throat deposition of fluticasone propionate** | | |
|---|---|---|
| **Comparison between tested product 125+25 µg and reference product 250+50 µg** | | |
| **Parameter** | **Mouth-throat deposition (IP +P) [µg]** | |
| Product | Tested product 125+25 µg | Reference product 250+50 µg |
| FP | 59.9 | 157.0 |

| | | |
|---|---|---|
| Reference product 250+50 µg, Mean for 3 batches, n = 3x10 = 30 (Air flow rate: 82 L/min), Tested product 125+25 µg, Mean for 3 batches, n = 3x10 = 30 (Air flow rate: 60 L/min), FP - fluticasone propionate, IP - Induction Port, P - Preseparator | | |

| **Mouth-throat deposition of salmeterol** | | |
|---|---|---|
| **Comparison between tested product 250+25 µg and reference product 500+50 µg** | | |
| **Parameter** | **Mouth-throat deposition (IP +P) [µg]** | |
| Product | Tested product 250+25 µg | Reference product 500+50 µg |
| S | 13.0 | 33.9 |

| | | |
|---|---|---|
| Reference product 500+50 µg, Mean for 3 batches, n = 3x10 = 30 (Air flow rate: 82 L/min), Tested product 250+25 µg, Mean for 3 batches, n = 3x10 = 30 (Air flow rate: 60 L/min), S - salmeterol, IP - Induction Port, P - Preseparator | | |

| **Mouth-throat deposition of fluticasone propionate** | | |
|---|---|---|
| **Comparison between tested product 250+25 µg and reference product 500+50 µg** | | |
| **Parameter** | **Mouth-throat deposition (IP +P) [µg]** | |
| Product | Tested product 250+25 µg | Reference product 500+50 µg |
| FP | 120.5 | 332.4 |

| | | |
|---|---|---|
| Reference product 500+50 µg, Mean for 3 batches, n = 3x10 = 30 (Air flow rate: 82 L/min), Tested product 250+25 µg, Mean for 3 batches, n = 3x10 = 30 (Air flow rate: 60 L/min), S - salmeterol, FP - fluticasone propionate, IP - Induction Port, P - Preseparator | | |

ND, FPF, FPD/ND comparison between tested product 125+25 µg and reference product 250+50 µg is presented below.

| **ND, FPF, FPD/ND comparison between tested product 125+25 µg and reference product 250+50 µg** | | | | | | |
|---|---|---|---|---|---|---|
| Parameter | ND [µg] | | FPF [%] | | FPD/ND [%] | |
| API | S | FP | S | FP | S | FP |
| Reference product 250+50 µg | 50 | 250 | 20,8 | 23,4 | 19,0 | 21,1 |
| Tested product 125+25 µg | 25 | 125 | 31,6 | 31,2 | 27,2 | 24,9 |

| | | | | | | |
|---|---|---|---|---|---|---|
| Reference product 250+50 µg, Mean for 3 batches, n = 3x10 = 30 (Air flow rate: 82 L/min), Tested product 125+25 µg, Mean for 3 batches, n = 3x10 = 30 (Air flow rate: 60 L/min), S - salmeterol, FP - fluticasone propionate, ND - nominal dose, FPF - fine particle fraction, FPD - fine particle dose | | | | | | |

ND, FPF, FPD/ND comparison between tested product 250+25 µg and reference product 500+50 µg is presented below.

| **ND, FPF, FPD/ND comparison between tested product 250+25 µg and reference product 500+50 µg** | | | | | | |
|---|---|---|---|---|---|---|
| Parameter | ND [µg] | | FPF [%] | | FPD/ND [%] | |
| API | S | FP | S | FP | S | FP |
| Reference product 500+50 µg | 50 | 500 | 20,1 | 21,2 | 18,4 | 19,4 |
| Tested product 250+25 µg | 25 | 250 | 31,9 | 30,5 | 27,6 | 25,0 |

| | | | | | | |
|---|---|---|---|---|---|---|
| Reference product 500+50 µg, Mean for 3 batches, n = 3x10 = 30 (Air flow rate: 82 L/min), Tested product 250+25 µg, Mean for 3 batches, n = 3x10 = 30 (Air flow rate: 60 L/min), S - salmeterol, FP - fluticasone propionate, ND - nominal dose, FPF - fine particle fraction, FPD - fine particle dose | | | | | | |

The following non-limiting examples are aimed at illustrating the invention.

### EXAMPLES

### Example 1

125/25 µg fluticasone propionate/salmeterol,
dry powder inhaler (*R_{D}* : 0.05 hPa^{0,5} min dm⁻³ to 0,15 hPa^{0,5} min dm⁻³).

The formulation was prepared of the components as follows:
Salmeterol (as xinafoate): 0.26% by weight (based on weight of salmeterol base)
Fluticasone propionate: 0.89% by weight
Mixture of lactose D90 < 250 µm: 98,85 % by weight
Mass of capsule content: 14 mg
The micronized active ingredients are mixed with lactose in high shear mixer for 12 minutes. Technical details of applied blending technology are well known and commonly used in pharmaceutical industry. The mixture is encapsulated into hard hydroxypropyl methylcellulose (HPMC) or gelatin capsules. Capsules filled with inhalation powder are packed into OPA/Alu/PVC//Alu blisters and then packed into carton boxes. The appropriate inhaler is attached to obtain the best inhalation system.

### Example 2

250/25 µg fluticasone propionate/salmeterol,
dry powder inhaler (*R_{D}* : 0.05 hPa^{0,5} min dm⁻³ to 0,15 hPa^{0,5} min dm⁻³).

The formulation was prepared of the components as follows:
Salmeterol (as xinafoate): 0.26% by weight (based on weight of salmeterol base)
Fluticasone propionate: 1.79% by weight
Mixture of lactose D90 < 250 µm: 97,95 % by weight
Mass of capsule content: 14 mg
The micronized active ingredients are mixed with lactose in high shear mixer for 12 minutes. Technical details of applied blending technology are well known and commonly used in pharmaceutical industry. The mixture is encapsulated into hard hydroxypropyl methyl cellulose or gelatin capsules. Capsules filled with inhalation powder are packed into OPA/Alu/PVC//Alu blisters and then packed into carton boxes. The appropriate inhaler is attached to obtain the best inhalation system.

### Example 3

250/25 µg fluticasone propionate/salmeterol,
dry powder inhaler (*R_{D}* : 0.05 hPa^{0,5} min dm⁻³ to 0,15 hPa^{0,5} min dm⁻³)

The formulation was prepared of the components as follows:
Salmeterol (as xinafoate): 0.23% by weight (based on weight of salmeterol base)
Fluticasone propionate: 1.56% by weight
Mixture of lactose D90 < 250 µm: 98,21 % by weight
Mass of capsule content: 16 mg
The micronized active ingredients are mixed with lactose in high shear mixer for 26 minutes. Technical details of applied blending technology are well known and commonly used in pharmaceutical industry. The mixture is encapsulated into hard hydroxypropyl methyl cellulose or gelatin capsules. Capsules filled with inhalation powder are packed into OPA/Alu/PVC//Alu blisters and then packed into carton boxes. The appropriate inhaler is attached to obtain the best inhalation system.

### Example 4

500/50 µg fluticasone propionate/salmeterol,
dry powder inhaler (*R_{D}* : 0.05 hPa^{0,5} min dm⁻³ to 0,15 hPa^{0,5} min dm⁻³)

A formulation was prepared of the components as follows:
Salmeterol (as xinafoate): 0.33% by weight (based on weight of salmeterol base)
Fluticasone propionate: 2.26% by weight
Mixture of lactose D90 < 250 µm: 97,41 % by weight
Mass of capsule content: 23 mg
The micronized active ingredients are mixed with lactose in high shear mixer for 48 minutes. Technical details of applied blending technology are well known and commonly used in pharmaceutical industry. The mixture is encapsulated into hard hydroxypropyl methyl cellulose or gelatin capsules. Capsules filled with inhalation powder are packed into OPA/Alu/PVC//Alu blisters and then packed into carton boxes. The appropriate inhaler is attached to obtain the best inhalation system.

## Claims

1. A pharmaceutical composition in the form of dry powder for inhalation comprising combination of two active pharmaceutical ingredients fluticasone propionate and salmeterol xinafoate for use in a method of treating asthma and chronic obstructive pulmonary disease (COPD), wherein particle size distribution of fluticasone propionate for d90 value is less than 10 µm, preferably less than 5 µm and particle size distribution of salmeterol xinafoate for d90 value is less than 10 µm, preferably less than 5 µm, **characterized in that** it is administered in a dose of 250/25 µg or 125/25 µg with a capsule inhaler having intrinsic aerodynamic resistance *R_{D}* in the range from 0.01 hPa^{0,5} min dm⁻³ to 0,2 hPa^{0,5} min dm⁻³.

2. A pharmaceutical composition of claim 1 having lactose as a carrier for the active pharmaceutical ingredients.

3. A pharmaceutical composition of claim 2 wherein the lactose is in the form of monohydrate.

4. A pharmaceutical composition according to claim 2 or 3, comprising lactose mixture of particle size distribution with d90 value of less than 250 µm.

5. A pharmaceutical composition according to any of claims 1-4, wherein the powder for inhalation is placed in hard capsules.

6. A pharmaceutical composition according to any of claims 1-5, wherein the formulation comprises not more than 1,5 % by weight of salmeterol xinafoate in total mass powder, and not more than 10 % by weight of fluticasone propionate in total mass powder, and not less than 90 % of carrier in total mass powder.

7. A pharmaceutical composition according to any of claims 1-6, to be administered by a capsule inhaler as a dosing device.

8. A pharmaceutical composition according to any of claims 5-7, wherein the powder for inhalation is placed in hard capsules of size 2, 3 or 4, preferably in size 3.

9. A pharmaceutical composition according to any of claims 5-8, wherein the main component of the capsule is gelatin or cellulose derivative, preferably hydroxypropyl methylcellulose.

10. A pharmaceutical composition according to any of claims 1-9, manufactured in a process comprising a stage of mixing the active pharmaceutical ingredients and carrier in a High Shear Mixer.

11. A set comprising one or more capsules with the powder composition of any of the preceding claims, and a capsule inhaler having intrinsic aerodynamic resistance R_{D} in the range from 0.01 hPa^{0,5} min dm⁻³ to 0.2 hPa^{0,5} min dm⁻³, preferably from 0.05 hPa^{0,5} min dm⁻³ to 0.15 hPa^{0,5} min dm⁻³, wherein the composition is administered by the inhaler in the dose of 250/25 µg or 125/25 µg.
